Europäisches Patentamt

European Patent Office

Office européen des brevets

(19)

(11) Veröffentlichungsnummer: **0 258 817 B1**

## (12) EUROPÄISCHE PATENTSCHRIFT

(45) Veröffentlichungstag der Patentschrift: **20.04.94**

(51) Int. Cl.5: **C07K 15/00**

(21) Anmeldenummer: **87112435.0**

(22) Anmeldetag: **27.08.87**

(54) Verwendung monoklonaler Antikörper zur Therapie von Tumoren.

(30) Priorität: **30.08.86 DE 3629640**

(43) Veröffentlichungstag der Anmeldung:
**09.03.88 Patentblatt 88/10**

(45) Bekanntmachung des Hinweises auf die
Patenterteilung:
**20.04.94 Patentblatt 94/16**

(84) Benannte Vertragsstaaten:
**AT BE CH DE ES FR GB GR IT LI LU NL SE**

(56) Entgegenhaltungen:
EP-A- 0 151 030          EP-A- 0 160 897
EP-A- 0 189 849          EP-A- 0 213 581
EP-A-01 410 79           DE-A- 3 329 184

THE LANCET, 15. März 1986, Nr. 8481, London, GB, R.W. BALDWIN et al."Monoclonal antibodies in cancer treatment"

(73) Patentinhaber: **BEHRINGWERKE Aktiengesellschaft**
**Postfach 1140**
**D-35001 Marburg(DE)**

(72) Erfinder: **Bosslet, Klaus, Dr.**
**Am Schlag 5**
**D-3550 Marburg(DE)**
Erfinder: **Schorlemmer, Hans Ulrich, Dr.**
**Am Kirschenwald 2**
**D-3550 Marburg(DE)**
Erfinder: **Sedlacek, Hans Harald, Dr.**
**Sonnenhang 3**
**D-3550 Marburg(DE)**

**Beschreibung**

Die Erfindung betrifft die Verwendung von monoklonalen Antikörpern (MAK) oder anderen Liganden, welche die Eigenschaft besitzen, sich an Pankreaskarzinom-Zellen zu binden und mindestens eine der zellulären Funktionen: Pinozytose von kolloidalem Gold, Produktion von Superoxidanion oder Freisetzung von Enzymen, besonders der neutralen Proteasen, ganz besonders Kollagenase oder Elastase, der Wachstumsfaktoren, Epidermal Growth Factor, Platelet Derived Growth Factor, Colony StimulatingFactor, Erythropoetin, Fibroblast Growth Factor, Tumor Angiogenesis Factor oder Transforming Growth Factor zu blockieren, für die Tumortherapie.

Zur Herstellung von solchen monoklonalen Antikörpern geeignete Verfahren sind in EP-A- 0 160 897, in der deutschen Offenlegungsschrift 33 29 184 und in der deutschen Patentanmeldung 35 31 301 (eingereicht 2.9. 1985) beschrieben. Als Liganden werden Moleküle bezeichnet, die keine monoklonalen Antikörper sind, jedoch ebenfalls an Pankreaskarzinom-Zellen binden und deren zellulären Funktionen hemmen, beispielsweise bestimmte Hormone.

Überraschenderweise wurde gefunden, daß humane Pankreaskarzinomzell-Linien lysosomale Enzyme ausscheiden, kolloidales Gold pinozytieren und Superoxidanion erzeugen können, und daß diese zellulären Funktionen durch eine Bindung von monoklonalen Antikörpern, insbesondere des 494/32, 495/36, 227/18 oder 227/19 an die Pankreaskarzinomzellen blockierbar sind. Die MAK 494/32 und 495/36 sind in der genannten Patentanmeldung beschrieben, während die übrigen in der genannten Offenlegungsschrift beschrieben sind. Sie sind dort folgendermaßen bezeichnet: 227/18 als AK 2 (Tabelle Seite 8) und 227/19 (AK 16).

Eine Konsequenz der Anlagerung solcher MAK mit der Folge einer Blockierung jener zellulären Funktionen sind Regressionen progressiv wachsender Pankreaskarzinome in Patienten.

Diese zellulären Funktionen von Pankreaskarzinom-Zellen schließen ein die Freisetzung besonders von neutralen Proteasen, ganz besonders Kollagenase oder Elastase, von Wachstumsfaktoren, Epidermal Growth Factor, Platelet Derived Growth Factor, Colony Stimulating Factor, Erythropoetin, Fibroblast Growth Factor, Tumor Angiogenesis Factor oder Transforming Growth Factor.

Monoklonale Antikörper (MAK), welche die Eigenschaften besitzen, sich an Pankreaskarzinom-Zellen zu binden und physiologische Funktionen, vorzugsweise die Pinozytose von kolloidalem Gold, die Superoxidanionproduktion oder die Freisetzung von Enzymen, besonders von neutralen Proteasen, ganz besonders Kollagenase oder Elastase, von Wachstumsfaktoren, Epidermal Growth Factor, Platelet Derived Growth Factor, Colony Stimulating Factor, Erythropoetin, Fibroblast Growth Factor, Tumor Angiogenesis Factor oder Transforming Growth Factor zu blockieren, sind demnach zur Therapie von Tumoren geeignet, deren Zellen diese zellulären Funktionen aufweisen. Ebenso geeignet sind bindende Fragmente eines solchen MAK, ausgenommen das Fc-Fragment, sowie andere Liganden, das heißt Moleküle mit der Eigenschaft an Pankreaskarzinom-Zellen zu binden und die genannten Funktionen zu blockieren.

Tumorzellen mit entsprechenden sekretorischen Eigenschaften sind neben Pankreaskarzinomen beispielweise Mammakarzinome, Ovarialkarzinome und Adenokarzinome der Lunge.

Gegenstand der Erfindung ist deshalb die Verwendung eines monoklonalen Antikörpers oder eines seiner Nicht Fc-Fragmente oder eines anderen Liganden, welche die Eigenschaft haben, folgende zelluläre Funktionen einer Tumorzelle: die Pinozytose von kolloidalem Gold, die Superoxidanionproduktion oder die Freisetzung von Enzymen, besonders der neutralen Proteasen, ganz besonders Kollagenase oder Elastase, der Wachstumsfaktoren, Epidermal Growth Factor, Platelet Derived Growth Factor, Colony Stimulating Factor, Erythropoetin, Fibroblast Growth Factor, Tumor Angiogenesis Facator oder Transforming Growth Factor von Pankreas-Tumorzellen zu blockieren, zur Therapie von Tumoren.

Mit solchen MAK oder anderen Liganden therapierbare Tumoren sind vorzugsweise Pankreaskarzinome, Mammakarzinome, Ovarialkarzinome oder Adenokarzinome der Lunge, besonders Pankreaskarzinome.

Zur Prüfung, ob die MAK oder Liganden an Pankreaskarzinom-Zellen binden, kann man Pankreaskarzinom-Zellinien, vorzugsweise die PANC-1-Linie (Int. J. Cancer (1975) 15, 741; ATCC CRL 1469) benutzen.

Zur Auswahl im erfindungsgemäßen Sinne verwendbarer MAK oder Liganden werden die Hemmung der Chemolumineszens von Zellen einer solchen Zellinie durch die MAK oder Liganden bestimmt. Die Bestimmung der Chemolumineszenz von Zellen ist eine Methode, die Erzeugung von Superoxidanion durch eine Zelle nachzuweisen und dessen Menge zu bestimmen.

Zu diesem Zweck wird die Tumorzell-Linie in "Dulbecco 's minimal essential medium" ("DMEM"), welches 100 ml/l fötales Kälberserum und 1 mmol/l Glutamin enthält (DMEM-FKS-GLN), kultiviert. Dazu wird sie in konfluentem Zustand bei 37° C für 1 Minute mit 2 g/l Trypsin und 0.2 g/l EDTA in "Puck's saline" behandelt und nach Waschen im Verhältnis von 1:20 in DMEM-FKS-GLN passagiert. Die vereinzelten Zellen werden bei einer Zellzahl von $10^6$ Zellen in einem Rundbodenröhrchen aus Polystyrol für 4 Stunden in DMEM adhärieren gelassen. Nach dreimaligem Waschen in DMEM werden 100 $\mu$l DMEM zugegeben und die Zellsuspensionen in die Zählkammer eines Biolumaten (LB 95 05 Berthold Co., Wildbad, FRG) gesetzt. Zum Inhalt des Röhrchens werden 50 $\mu$l Luminol (100 $\mu$g/ml) und 100 $\mu$l eines Stimulus (50 $\mu$g/ml Zymosan oder 100 $\mu$g/ml eines Immunkomplexes oder zur Kontrolle 100 $\mu$l PBS) gegeben und die Lichtemission gemessen (Apple II Computer mit MX-82 FIT Epson dot matrix printer). Zur Bestimmung der Inhibition durch einen MAK wird 100 $\mu$l einer Lösung des MAK zugegeben und 15 Minuten inkubiert (Endkonzentration 10 $\mu$g/ml).

Die MAK 494/32, 495/36, 227/18, 431/31 sowie die weiteren in der DOS 33 29 184 beschriebenen 227/12 (AK 12) und 227/19 (AK 16) wurden beispielhaft in dieses oder die folgenden Testsysteme eingesetzt sowie ihre Bindung an die entsprechenden Tumorzell-Linien im indirekten Immunfloreszenzassay nach Terasaki (Cancer detection and prevention (1983) 6, 181 gemessen.

Die folgende Tabelle 1 zeigt die Ergebnisse.

## Tabelle 1

| MAK | Bindungs-test (Terasaki) | Inhibition der Chemolumineszenz(%) Stimulus | | |
|---|---|---|---|---|
| | | PBS (Kontrolle) | Zymosan | Immunkomplex |
| 227/12 | negativ | 0 | 7 | 3 |
| 431/31 | negativ | 0 | 8 | 0 |
| 494/32 | positiv | 6 | 59 | 37 |
| 227/18 | positiv | 21 | 54 | 66 |
| 227/19 | positiv | 17 | 42 | 48 |

Aus der Tabelle ist ersichtlich, daß die sich nicht an die Tumorzellmembran bindenden MAK die Chemolumineszenz nicht inhibieren, während die bindenden inhibieren.

Dies trifft ebenfalls für andere MAK zu, die als Tumortherapeutika geeignet sind.

Zur Prüfung auf die Fähigkeit eines MAK die Pinozytose und Enzymsekretion zu hemmen, wurde folgendermaßen verfahren:

$3 \times 10^6$ wie oben beschrieben erhaltene und in 3.5 cm Petrischalen kultivierte Tumorzellen werden für 24 Stunden mit den Stimuli (Zymosan oder ein Immunkomplex oder PBS (Kontrolle) und gegebenenfalls zusammen mit 10 $\mu$g MAK/ml) inkubiert, und die Fähigkeit de MAK zur Inhibierung der Enzymfreisetzung der Zellen entsprechend den angegebenen Literaturstellen geprüft ($\beta$-Glucuronidase: J. biol. Chem. (1946) 166, 757; $\beta$-galactosidase: Biochem. J. (1959) 71, 318; Laktatdehydrogenase: Methoden der enzymatischen Analyse , Verlag Chemie, Weinheim/Bergstraße, Bundesrepublik Deutschland, Seite 533 (1970); Pinozytotische Aktivität: Biochem. Biophys. Res. Comm. (1973) 52, 627).

In Tabelle 2 ist ebenfalls gezeigt, daß MAK, die an die PANC-1-Zelle binden, auch deren Enzymfreisetzung inhibiert, während andere dies nicht bewirken.

Tabelle 2

Enzymfreisetzung ( % Inhibition)

| MAK | PBS | Zym | IC |
|---|---|---|---|
| 494/32 | 21 | 42 | 34 |
| 495/36 | 26 | 86 | 90 |
| 227/18 | 2 | 52 | 52 |
| 227/19 | 0 | 54 | 51 |
| 431/31 | 4 | 7 | 3 |
| 227/12 | 5 | 0 | 0 |

In Tabelle 3 ist die Fähigkeit von monoklonalen Antikörpern dargestellt, die basale pinozytotische Aktivität von Pankreastumorzellen nach Bindung an die Tumorzellmembran zu inhibieren. Die nichtbindenden inhibieren die Pinozytose von $^{198}$ Au nicht. Identische Befunde wie mit intaktem Immunglobulin wurden auch mit dem F(ab')$_2$-Fragment des MAK 494/32 erhalten.

Tabelle 3

| MAK | Inhibition der Aufnahme von $^{198}$ Au (%) |
|---|---|
| 494/32 | 42 |
| 495/36 | 53 |
| 227/18 | 61 |
| 227/19 | 60 |
| 431/31 | 5 |
| 227/12 | 4 |

Mak oder deren nicht Fc-Fragmente oder andere Liganden, die in der Lage sind, alle oder einige der oben angegebenen Pankreaskarzinomzellfunktionen zu inhibieren, können zur Therapie von Tumoren, besonders von Pankreaskarzinomen verwendet werden.

Klinische Daten zeigen nach i.v. Applikation des MAK 494/32 (5x in eintägigen Abständen bis 210 mg Gesamtdosis), daß dieser MAK nach palliativer Operation bei progressivem Pankreaskarzinom in 4 von 6 Fällen einen Stillstand der Erkrankung (computertomographisch gesichert) und eine subjektive Besserung des Allgemeinbefindens der Patienten bewirkt.

Ein zur Therapie der Tumoren geeignetes Arzneimittel enthält einen MAK oder Liganden mit den beschriebenen Eigenschaften in Form einer Lösung, in gefrorener oder getrockneter Form. Eine Tagesdosis liegt im Bereich von 20 bis 500 mg pro 70 kg Körpergewicht. Das Mittel wird parenteral angewendet, vorzugsweise infundiert. Eine wirksame Menge wird in einem Zeitraum von einer Minute bis 3 h dem Patienten verabreicht. Das Mittel wird in Zeitintervallen von einem Tag bis zu mehreren Monaten gegeben.

EP 0 258 817 B1

**Patentansprüche**

1. Verwendung eines monoklonalen Antikörpers, der an definierte zytoplasmatische, mebranassoziierte oder sezernierte Tumorantigene bindet, oder eines seiner Nicht-Fc-Fragmente oder eines Liganden, zur Herstellung eines Arzneimittels oder Diagnostikums, das die Pinozytose von kolloidalem Gold, die Superoxidanionproduktion oder die Freisetzung von Enzymen, besonders von neutralen Proteasen, ganz besonders Kollagenase oder Elastase, von Wachstumsfaktoren, Epidermal Growth Factor, Platelet Derived Growth Factor, Colony Stimulating Factor, Erythropoietin, Fibroblast Growth Fastor, Tumor Angiogenesis Factor oder Transforming Growth Factor von Pankreastumorzellen, Mammakarzinomzellen, Ovarialkarzinomzellen oder Adenokarzinomzellen der Lunge blockiert.

2. Verwendung eines monoklonalen Antikörpers gemäß Anspruch 1, dadurch gekennzeichnet, daß der monoklonale Antikörper ein Epitop auf einem Glykoprotein von einem Molekulargewicht von über 200 kDa unter nicht-reduzierenden oder unter reduzierenden Bedingungen, welches Epitop sowohl resistent gegen Formaldehydfixierung oder Paraffineinbettung als auch gegen Neuraminidasebehandlung ist und durch Perjodatoxidation zerstört wird, oder ein Epitop auf einem Glykoprotein von einem Molekulargewicht von über 200 kDa unter nicht-reduzierenden oder unter reduzierenden Bedingungen, welches Epitop sowohl gegen Neuraminidasebehandlung als auch gegen Perjodatoxidation resistent, jedoch gegen Formaldehydbehandlung oder Paraffineinbettung nicht resistent ist, erkennt.

3. Verwendung eines Liganden gemäß Anspruch 1, dadurch gekennzeichnet, daß der Ligand die Freisetzung der Urokinase blockiert.

4. Verwendung eines monoklonalen Antikörpers gemäß Anspruch 1, dadurch gekennzeichnet, daß der monoklonale Antikörper MAK 494/32 ist.

5. Verwendung eines monoklonalen Antikörpers gemäß Anspruch 1, dadurch gekennzeichnet, daß der monoklonale Antikörper MAK 495/36 ist.

6. Verwendung eines monoklonalen Antikörpers gemäß Anspruch 1, dadurch gekennzeichnet, daß der monoklonale Antikörper MAK 227/18 (AK 2) ist.

7. Verwendung eines monoklonalen Antikörpers gemäß Anspruch 1, dadurch gekennzeichnet, daß der monoklonale Antikörper MAK 227/19 (AK 16) ist.

**Claims**

1. The use of a monoclonal antibody which binds to defined cytoplasmic, membrane-associated or secreted tumor antigens, or of one of its non-Fc fragments or of a ligand, for the production of a pharmaceutical or diagnostic aid which blocks the pinocytosis of colloidal gold, the production of superoxide anion or the release of enzymes, especially of neutral proteases, very especially collagenase or elastase, of growth factors, epidermal growth factor, platelet derived growth factor, colony stimulating factor, erythropoietin, fibroblast growth factor, tumor angiogenesis factor or transforming growth factor from pancreatic tumor cells, breast carcinoma cells, ovarian carcinoma cells or adenocarcinoma cells of the lung.

2. The use of a monoclonal antibody as claimed in claim 1, wherein the monoclonal antibody recognises an epitope on a glycoprotein of a molecular weight of above 200 kDa under non-reducing or under reducing conditions, which epitope is both resistant to formaldehyde fixation or paraffin embedding and to neuraminidase treatment and is decomposed by periodate oxidation, or an epitope on a glycoprotein of a molecular weight of above 200 kDa under non-reducing or under reducing conditions, which epitope is resistant both to neuraminidase treatment and to periodate oxidation but is not resistant to formaldehyde treatment or paraffin embedding.

3. The use of a ligand as claimed in claim 1, wherein the ligand blocks the release of urokinase.

4. The use of a monoclonal antibody as claimed in claim 1, wherein the monoclonal antibody is MAb 494/32.

**5.** The use of a monoclonal antibody as claimed in claim 1, wherein the monoclonal antibody is MAb 495/36.

**6.** The use of a monoclonal antibody as claimed in claim 1, wherein the monoclonal antibody is MAb 227/18 (Ab 2).

**7.** The use of a monoclonal antibody as claimed in claim 1, wherein the monoclonal antibody is MAb 227/19 (Ab 16).

**Revendications**

**1.** Utilisation d'un anticorps monoclonal qui se lie à des antigènes tumoraux cytoplasmiques définis, associés au membranes ou sécrétés, ou d'un fragment non Fc de celui-ci, ou d'un ligand, pour la fabrication d'un médicament ou d'un agent de diagnostic, qui bloque la pinocytose de l'or colloïdal, la production d'anion superoxyde ou la libération d'enzymes, en particulier de protéases neutres, tout particulièrement de la collagénase ou de l'élastase, des facteurs de croissance, du facteur de croissance épidermique, du facteur de croissance dérivé des plaquettes, du facteur de stimulation de colonies, de l'érythropoïétine, du facteur de croissance des fibroblastes, du facteur d'angiogénèse tumorale ou du facteur transformant de croissance de cellules tumorales pancréatiques, de cellules du cancer du sein, de cellules du cancer de l'ovaire ou de cellules d'adénocarcinome du poumon.

**2.** Utilisation d'un anticorps monoclonal selon la revendication 1, caractérisé en ce que l'anticorps monoclonal reconnaît un épitope sur une glycoprotéine ayant une masse moléculaire de plus de 200 kd, dans des conditions non réductrices ou dans des conditions réductrices, lequel épitope est résistant aussi bien à la fixation par le formaldéhyde ou à l'incorporation dans la paraffine qu'au traitement par la neuraminidase, et est décompose par l'oxydation au périodate, ou un épitope sur une glycoprotéine ayant un masse moléculaire de plus de 200 kd, dans des conditions non réductrices ou dans des conditions réductrices, lequel épitope est résistant aussi bien au traitement à la neuraminidase qu'à l'oxydation par le periodate, mais n'est pas résistant au traitement par le formaldéhyde ni à l'incorporation dans la paraffine.

**3.** Utilisation d'un ligand selon la revendication 1, caractérisé en ce que le ligand bloque la libération de l'urokinase.

**4.** Utilisation d'un anticorps monoclonal selon la revendication 1, caractérise en ce eue l'anticorps monoclonal est l'AcM 494/32.

**5.** Utilisation d'un anticorps monoclonal selon la revendication 1, caractérisé en ce que l'anticorps monoclonal est l'AcM 495/36.

**6.** Utilisation d'un anticorps monoclonal selon la revendication 1, caractérisé en ce que l'anticorps monoclonal est l'AcM 227/18 (AK 2).

**7.** Utilisation d'un anticorps monoclonal selon la revendication 1, caractérisé en ce que l'anticorps monoclonal est l'AcM 227/19 (AK 16).